Europäisches Patentamt

European Patent Office

Office européen des brevets

(19)

(11) Publication number: **0 329 164 B1**

# EUROPEAN PATENT SPECIFICATION

(12)

(45) Date of publication of patent specification: **28.09.94**

(51) Int. Cl.⁵: **C12N 1/20**, C12N 1/04, A23K 3/00, //(C12N1/20, C12R1:225,1:46)

(21) Application number: **89102777.3**

(22) Date of filing: **17.02.89**

(54) **Lactic acid bacteria starter for preparation of silage and agent containing the bacteria in the viable state.**

(30) Priority: **18.02.88 JP 33961/88**
**16.05.88 JP 117094/88**

(43) Date of publication of application:
**23.08.89 Bulletin 89/34**

(45) Publication of the grant of the patent:
**28.09.94 Bulletin 94/39**

(84) Designated Contracting States:
**DE FR GB**

(56) References cited:
**DE-A- 245 271**

(73) Proprietor: **THE CALPIS FOOD INDUSTRY CO., LTD.**
**20-3, Ebisu-Nishi 2-chome**
**Shibuya-ku Tokyo 150 (JP)**

(72) Inventor: **Minoru, Shibata**
**c/o Zenkoku Rakunogyo**
**Kyodokumiai Rengokai**
**Chikusankaikan,4-9-2 Ginza,Chuo-ku,Tokyo**
**(JP)**

Inventor: **Hiroshi, Kubozono**
**c/o Zenkoku Rakunogyo**
**Kyodokumiai Rengokai**
**Chikusankaikan,4-9-2 Ginza,Chuo-ku,Tokyo**
**(JP)**
Inventor: **Syozi, Kawanabe**
**c/o Zenkoku Rakunogyo**
**Kyodokumiai Rengokai**
**Chikusankaikan,4-9-2 Ginza,Chuo-ku,Tokyo**
**(JP)**
Inventor: **Takefumi, Iwanami c/o The Calpis Food Ind. Co.Ltd**
**Research and Development Center**
**2-4-1**
**Ebisu Minami Shibuya-ku, Tokyo (JP)**
Inventor: **Kiyoshi, Maruta c/o The Calpis Food Ind. Co. Ltd**
**Research and Development Center**
**2-4-1**
**Ebisu Minami Shibuya-ku, Tokyo (JP)**

EP 0 329 164 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Ichiya, Murota c/o The Calpis Food Ind. Co. Ltd**
**Research and Development Center**
**2-4-1**
**Ebisu Minami Shibuya-ku, Tokyo (JP)**
Inventor: **Hiroshi, Miyazaki c/o The Calpis Food Ind.Co.Ltd**
**Research and Development Center**
**2-4-1**
**Ebisu Minami Shibuya-ku, Tokyo (JP)**

(74) Representative: **Wehnert, Werner, Dipl.-Ing.**
**Patentanwälte**
**Dipl.-Ing. Graalfs,**
**Dipl.-Ing. Hauck,**
**Dipl.-Ing. Wehnert,**
**Dr.-Ing. Döring**
**Mozartstrasse 23**
**D-80336 München (DE)**

**Description**

BACKGROUND OF THE INVENTION

This invention relates to a lactic acid bacteria starter and an agent or medicine containing the bacteria in the viable state. More particularly, it relates to a lactic acid bacteria starter for preparation of silage having a high rate of survival of the lactic acid bacteria and allowing to produce silage of higher quality, and the agent or medicine containing the bacteria in the viable state.

In general, for preparing high quality silage, it is essential that fermentation of lactic acid take place thoroughly at the initial stage to lower the pH promptly. However, in effect, sufficient fermentation does not necessarily occur since it depends on the temperature or on the species or water contents of the plants from which the silage is prepared. Therefore, for preparing the silage of higher quality, it is advisable not only to resort to the lactic acid bacteria naturally affixed to the plants, but to add live or viable lactic acid bacteria and/or an agent containing the bacteria in the viable state suitable for silage fermentation artificially and at a higher concentration to effect stable lactic acid fermentation. Although a number of viable lactic acid bacteria and agents containing the lactic acid bacteria in the live or viable state have been studied and inspected, the situation is that a satisfactory lactic acid bacteria starter has not yet been obtained.

On the other hand, while a sufficient quantity of the live or viable lactic acid bacteria exist at the time of preparation of the agent containing the viable lactic acid bacteria, these live bacteria die gradually in the course of the circulation in the market, such that a sufficient quantity of the live lactic acid bacteria is not available when the agent is actually employed for preparation of silage. Also, the current situation is that, even if the sufficient quantity of the live lactic acid bacteria is available at this time, the produced agent containing the live lactic acid bacteria is not in the state in which it can be added easily to the silage plants and hence it cannot be added uniformly to the silage plants.

SUMMARY OF THE INVENTION

It is a principal object of the present invention to provide a lactic acid bacteria starter for preparation of silage having homogeneous quality which is rich in flavor and liked by the livestock, such as cattle, and an agent containing the bacteria in the live state.

It is another object of the present invention to provide a lactic acid bacteria starter for preparation of silage having an extremely high rate of survival or viability of the lactic acid bacteria, and an agent containing the bacteria in the live state.

It is still another object of the present invention to provide a lactic acid bacteria starter for preparation of silage that can be added uniformly to the silage.

The above and other objects of the invention will become apparent from the following description.

In accordance with the present invention, there is provided a lactic acid bacteria starter for preparation of silage comprising a strain Lactobacillus casei C-1631 as the effective bacteria.

In accordance with the present invention, there is also provided a live lactic acid bacteria-containing agent for preparation of silage comprising the above lactic acid bacteria starter for preparation of silage containing a strain Lactobacillus casei C-1631 as the effective bacteria and a base containing not less than 50% by weight of corn grits having a moisture content of not more than 4% by weight and a particle size of 10 to 80 meshes.

In accordance with the present invention, there is also provided a lactic acid bacteria starter for preparation of silage comprising a strain Lactobacillus casei C-1631 and a strain Streptococcus lactis C-6513 as the effective bacteria.

In accordance with the present invention, there is also provided a live lactic acid bacteria-containing agent for preparation of silage comprising the aforementioned lactic acid bacteria starter containing the strain Lactobacillus casei C-1631 and the strain Streptococcus lactis C-6513 as the effective bacteria, and the aforementioned base.

PREFERRED EMBODIMENTS OF THE INVENTION

The present inventors sorted out lactic acid bacteria necessary for preparation of high flavor silage in the homogeneous state and conducted eager researches on these bacteria. As a result of these researches, it has been found that silage extremely rich in flavor can be prepared in the homogeneous state by using a novel strain Lactobacillus casei C-1631 alone or both strains Lactobacillus casei C-1631 and streptococcus lactis C-6513 as the effective bacteria.

The two strains of species as the effective bacteria employed in the present invention are Lactobacillus casei C-1631 and Streptococcus lactis C-6513 newly isolated by the present inventors from nature. Specifically, silage prepared in Nasu City, Tochigi Prefecture, Japan was applied to a BL agar medium produced by Nisui Seika KK and was then cultivated to obtain colonies, which were picked up and classified by "Bergey's Manual of Determinative Bacteriology" Eighth Edition, 1974, by R.E. Buchanan & N.E. Gibbons, The Williams & Wilkins Company. The thus identified strain was named Lactobacillus casei C-1631. Alternatively, the silage from the same place was applied to a Trypticase Soy agar medium. The same procedures as mentioned above were followed to obtain Streptococcus lactis C-6513.

The strain Lactobacillus casei C-1631, deposited with Fermentation Reseach Institute of the Agency of Industrial Science and Technology under the domestic deposition number 9809 and the international deposition No. BP-2260, has the following bacteriological properties:

A. Microbiological Properties

(1) bacillus (0.3 to 0.6 $\mu$m in width and 1.5 to 2.5 $\mu$m in length);
(2) gram stain, positive;
(3) formation of spores, none;
(4) mobility, none;
(5) facultative anaerobic;

B. States of Growth on Various Media

(1) On a APT liquid medium (Difco): Very good growth.
(2) On a BL agar medium(anaerobic cultivation at 30°C for three days): the colony is a grayish rough colony having an orange color at the center and yellow to green to gray color on the periphery.

C. Physiological Properties

(1) $CO_2$ production from glucose: -
(2) Production of catalase: -
(3) Reducibility of nitrates: -
(4) Liquefaction of gelatine: -
(5) Production of $H_2S$: -
(6) Production of indole: -
(7) Aerobic growth: +
(8) Growth at 15 °C: +
(9) Growth at 45°C: +
(10) Optical activity of lactic acid: L (+)
(11) Fermentation of Carbohydrate
arabinose: -
xylose: -
rhamnose: -
glucose: +
mannose: +
fructose: +
galactose: +
sucrose: +
maltose: +
cellobiose: +
lactose: -
trehalose: +
melibiose: -
raffinose: -
starch:-
mannitol: +
sorbitol: +
esculin: +
salicin: +

The strain Streptococcus lactis C-6513 has been deposited with Fermentation Reseach Institute of the Agency of Industrial Science and Technology under the domestic deposition number 9810 and the international deposition No. BP-2261, and has the following bacteriological properties:

A. Microbiological Properties

(1) coccus (1.0 to 1.5 $\mu$m in diameter);
(2) gram stain, positive;
(3) formation of spores, none;
(4) mobility, none;
(5) facultative anaerobic;

B. State of Growth on Various Media

(1) On Trypticase Soy Liquid Medium (BBL): Very good growth.
(2) State of colony on a BL agar medium (anaerobic cultivation at 30°C for three days): A smooth colony of grayish color.

C. Physiological Properties

(1) $CO_2$ production from glucose: -
(2) Production of catalase: -
(3) Growth on 0.1% methylene blue milk: +
(4) Growth on 6.5% NaCl: -
(5) Growth in 40% bile: +
(6) Growth at pH of 9.6: -
(7) Viability after processing at 60°C for 30 minutes: -
(8) Hemolytic Properties for horse's blood: -
(9) Liquefaction of gelatine: -
(10) Hydrolysis of starch: -
(11) Decomposition of sodium hippurate: -
(12) Hydrolysis of esculin: -
(13) Production of $NH_3$ from arginine: +
(14) Growth at 10°C: +
(15) Growth at 45°C: -
(16) Fermentation of Carbohydrate
arabinose: +
xylose: +
rhamnose: -
glucose: +
mannose: +
fructose: +
galactose: +
sucrose: +
maltose: +
cellobiose: +
lactose: +
trehalose: +
melibiose: -
raffinose: -
mannitol: +
sorbitol: -
salicin: +

In the method of cultivation of the aforementioned two strains of species employed in the present invention, culture media commonly employed for cultivation of microorganisms including compounds selected from the group consisting of, for example, a carbon source, a nitrogen source, inorganic substances, vitamins, amino acids or a mixture thereof, may be employed. As the aforementioned carbon source, any assimilable carbon compound selected from the group consisting of, for example, glucose, sucrose, maltose and a mixture thereof, may be employed. As the aforementioned nitrogen source, any available nitrogen compounds selected from the group consisting of, for example, peptone, meat extracts, casein hydrolysates and a mixture thereof, may be employed. If necessary, an additive selected from the group consisting of salts such as phosphates, salts of magnesium, sodium, potassium, iron or manganese, vitamins, amino acids, defoaming agents and surfactants, may be employed in the culture medium, either alone or in combination.

The culture medium may be either liquid or solid. The initial pH of the medium is 5 to 8 and preferably 6 to 8 and the cultivation temperature is 20 to 42°C and preferably 25 to 40°C. The cultivation time is 12 hours to three days and preferably 15 to 24 hours. The cultivation may be performed separately for the strains of species, or simultaneously for a mixture of the two strains of species if the two stains are used.

The culture product obtained in accordance with the present invention may be employed as the lactic acid bacteria starter for preparation of silage either directly or in the form of separate bacteria or separated and washed bacteria. The culture product or the bacteria may be dried and formed into an agent with or without addition of additives.

In the preparation of the live lactic acid bacteria-containing agent for silage preparation, a base containing not less than 50 wt.% of corn grits having the moisture content of not more than 4 wt.% and the particle size of 10 to 80 meshes, is preferably employed. The corn grits mean in general corn powders obtained upon roughly grinding the corn. According to the present invention, commercially available corn grits are extensively employed. However, since these commercially available corn grits are high in moisture contents, it is necessary to dry the grits to moisture contents of not more than 4 wt.% with the use of a drier. The moisture contents in the corn grits affect the viability of the lactic acid bacteria significantly. The moisture contents in the corn grits in excess of 4 wt.% are not desirable since then the corn grits can no longer be used because the viability of the lactic acid bacteria in the produced agent is drastically lowered.

The particle size of the corn grits is an important factor affecting the dispersibility and viability of the lactic acid bacteria. The particle size of the corn grits in excess of 10 meshes is not desirable since the live lactic acid bacteria and the corn grits tend to be separated from each other due to the coarse particle size, such that, even when the agent containing the live lactic acid bacteria is scattered uniformly, the lactic acid bacteria contained in the live lactic acid bacteria-containing agent are not necessarily added uniformly to the silage. The particle size of the corn grits less than 80 meshes is also not desirable since the operation of adding the live lactic acid bacteria-containing agent for preparation of silage is usually performed in the field and hence the corn grits of such small size tends to fly in the wind to interfere with uniform mixing of the agent with the plants used for preparation of silage.

It suffices that the base contains not less than 50 wt.% of the corn grits. Although the base consisting only of the corn grits may be employed, less than 50 wt.% of an additive selected from the group consisting of thoroughly dried calcium carbonate, zeolite, corn cob, dextrin and a mixture thereof, may be employed in the base.

The live lactic acid bacteria-containing agent used for preparation of silage may be prepared by uniformly mixing the culture product of the aforementioned effective bacteria and the base in a V-type mixer, such as a ribbon mixer.

In usage, the lactic acid bacteria starter and the agent containing the live lactic acid bacteria may be added to the silage plants so that the number of the aforementioned effective bacteria amounts to $10^2$ to $10^8$ and desirably to $10^4$ to $10^7$ per gram of the silage plants.

The lactic acid bacteria starter for preparation of silage and the agent containing the live lactic acid bacteria according to the present invention enjoy an extremely high rate of survival of the lactic acid bacteria and can be added uniformly to the silage plants so that there may be obtained a silage having high flavor and liked by the livestock, such as cattle.

EXAMPLES OF THE INVENTION

The present invention will be explained hereinbelow in more detail with reference to Synthesis Examples and Examples. It should be noted that these Examples are given by way of illustration only and are not intended for limiting the present invention.

Synthesis Example 1

200g of casein peptone, 30g of yeast extract, 20 g of bipotassium phosphate and 50g of glucose were dissolved in 10 liters of deionized water to produce a culture medium, the pH of which was then adjusted to 7.5 by an aqueous solution of sodium hydroxide. The culture medium thus prepared was placed in a jar fermentor, sterilized at 121 °C for 15 minutes and allowed to cool. A liquid culture of previously incubated strain Lactobacillus casei C-1631, FERM P-9809 (BP-2260), was inoculated to the medium and allowed to stand for incubation at 35 °C for 20 hours.

The thus produced liquid culture was centrifuged to collect bacteria which were then freeze dried using a pre-sterilized 10% defatted milk - 1% sodium glutamate solution as the dispersion medium to produce 450 g of a live bacteria-containing agent containing the strain Lactobacillus casei C-1631. The number of the live bacteria contained in the live bacteria-containing agent was $1 \times 10^{10}$/gram.

Synthesis Example 2

A culture medium was prepared as in Synthesis Example 1 and inoculated with a previously incubated liquid culture of the strain Streptococcus lactis C-6513, FERM P-9810 (BP-2261) and the cultivation was continued stationarily at 30 °C for 18 hours.

The thus produced liquid culture was centrifuged to collect bacteria which were then freeze dried using the 10% defatted milk as the dispersion medium to produce 410g of a live bacteria-containing agent containing the strain Streptococcus lactis C-6513. The number of the live bacteria containing in the live bacteria-containing agent was $1 \times 10^{10}$/gram.

Synthesis Example 3

A culture medium was prepared as in Synthesis Example 1 and inoculated with a previously incubated liquid culture of the strains of Lactobacillus casei C-1631, FERM P-9809 (BP-2260) and Streptococcus lactis C-6513, FERM P-9810 (BP-2261) and the concurrent cultivation was performed stationarily at 30 °C for 18 hours.

The thus produced liquid culture was centrifuged to collect bacteria which were then freeze dried using a 10% defatted milk - 1% sodium glutamate solution as the dispersion medium to produce 490g of a mixed live bacteria-containing agent containing Lactobacillus casei C-1631 and Streptococcus lactis C-6513. The number of live Lactobacillus casei C-1631 bacteria and the number of live Streptococcus lactis C-6513 bacteria contained in the live bacteria-containing agent were $1 \times 10^{10}$ and $6 \times 10^{9}$, respectively.

Synthesis Example 4

To 1 part of dried bacteria obtained in Synthesis Example 1 were added 9 parts of wheat bran and the resulting mass was mixed thoroughly to produce a live bacteria-containing agent. The number of the live bacteria contained in the agent was $8 \times 10^{8}$/gram.

Synthesis Example 5

To 1 part of dried bacteria obtained in Synthesis Example 2 were added 9 parts of calcium carbonate and the resulting mass was mixed thoroughly to produce a live bacteria-containing agent. The number of the live bacteria contained in the agent was $7 \times 10^{8}$/gram.

Synthesis Example 6

To 1 part of the dried bacteria obtained in Synthesis Example 3 were added 9 parts of corn starch and the resulting mass was mixed thoroughly. The number of live Lactobacillus casei C-1631 bacteria and the number of live Streptococcus lactis C-6513 bacteria contained in the agent were $8 \times 10^{8}$/g and $5 \times 10^{8}$/g, respectively.

Synthesis Example 7

20kg of corn grits having a particle size of 32 to 60 meshes were spread to a thin thickness of a tray and dried at 100 °C for four hours by a ventilated shelf type drier. The water contents of the dried corn grits were found to be 3.0% (w/w).

Synthesis Example 8

20kg of corn grits having a particle size of 35 to 80 meshes were spread to a thin thickness of a tray and dried at 100°C for five hours by a ventilated shelf type drier. The water contents of the dried corn grits were found to be 2.7% (w/w).

Synthesis Example 9

20kg of corn grits having a particle size of 10 to 32 meshes were spread to a thin thickness on a tray and dried at 100°C for three hours by a ventilated shelf type drier. The water contents of the dried corn grits were found to be 4.0% (w/w).

Synthesis Example 10-1 ( Control Example)

To 1 part of the dried bacteria obtained in Synthesis Example 1 were added 9 parts of commercially available corn grits and the resulting mass was mixed thoroughly to produce a live bacteria-containing agent. The number of live lactic acid bacteria contained in the agent immediately after its preparation was 8 X $10^8$/g.

Synthesis Example 10-2 (Control Example )

To 1 part of the dried bacteria obtained in Synthesis Example 1 were added 9 parts of wheat bran and the resulting mass was mixed thoroughly to produce a live bacteria-containing agent. The number of live lactic acid bacteria contained in the agent immediately after its preparation was 8 X $10^8$/g.

Synthesis Example 11

To 1 part of the dried bacteria obtained in Synthesis Example 1 were added 9 parts of the corn grits prepared in the Synthesis Example 7 and the resulting mass was mixed thoroughly to produce a live bacteria-containing agent. The number of live lactic acid bacteria contained in the agent immediately after its preparation was 9 X $10^8$/g.

Synthesis Example 12 ( Control Example )

To 1 part of the dried bacteria obtained in Synthesis Example 2 were added 5 parts of commercially available corn grits, 2 parts of calcium carbonate and 2 parts of zeolite and the resulting mass was mixed thoroughly to produce a live bacteria-containing agent. The number of the live lactic acid bacteria contained in the agent immediately after its preparation was 8 X $10^8$/g.

Synthesis Example 13

To 1 part of the dried bacteria obtained in Synthesis Example 2 were added 5 parts of the corn grits obtained in Synthesis Example 8, 2 parts of calcium carbonate and 2 parts of zeolite and the resulting mass was mixed thoroughly to produce a live bacteria-containing agent. The number of the live lactic acid bacteria contained in the agent immediately after its preparation was 8 X $10^8$/g.

Synthesis Example 14 ( Control Example )

To 1 part of the dried bacteria obtained in Synthesis Example 3 were added 7 parts of commercially available corn grits and 2 parts of corn cob and the resulting mass was mixed thoroughly to produce a live bacteria-containing agent. The number of the live L. casei C-1631 and S. lactis C-6513 contained in the agent immediately after its preparation was 8 X $10^8$/g and 4 X $10^8$/g, respectively.

Synthesis Example 15

To 1 part of the dried bacteria obtained in Synthesis Example 3 were added 7 parts of the corn grits obtained in Synthesis Example 9 and 2 parts of dried corn cob and the resulting mass was mixed thoroughly to produce a live bacteria-containing agent. The number of the live L. casei C-1631 and S. lactis C-6513 contained in the agent immediately after its preparation was $9 \times 10^8$/g and $5 \times 10^8$/g, respectively.

Example 1

Reaped orchard grass, reed Canarygrass, Italian ryegrass and alfalfa were dried for half a day in the sun, shredded and mixed to give half dried grass. To 700g of the half dried grass were added 0.5g of each of the live bacteria-containing agents obtained in the Synthesis Examples 4 and 5 to give a dual agent group -1. Similarly, to 700g of the half dried grass was added 1g of the live bacteria-containing agent obtained in Synthesis Example 6 to give a dual agent group -2. Each of the groups -1 and -2 was packed under pressure into a capped polyethylene vessel and aged at 30°C for 22 days to produce silage. After test samples were taken out, the silage was further aged at 37°C for ten days. A test group was also prepared in which the strain Lactobacillus casei C-1631 produced by the Synthesis Example 4 by using the same ratio was added to the half dried grass as a sole agent group. For comparison, a control group in which the live bacteria-containing agent was not added to the half dried grass was prepared.

The results are shown in Table 1.

The pH values were measured as to the samples of filtrate of 10 w/v% extraction liquids of the shredded silage (5°C, 24 hours). The organic acids were analyzed quantitatively by gas chromatography of the above samples.

Table 1

| Aging Conditions | | pH | Lactic acid (%) | Butyric acid (%) |
|---|---|---|---|---|
| At 30°C for 22 days | Dual Agent Group-1 | 4.2 | 3.9 | 0 |
| | Dual Agent Group-2 | 4.1 | 4.0 | 0 |
| | Sole Agent Group | 4.4 | 3.1 | 0 |
| | Control Group | 4.6 | 1.7 | 0.3 |
| At 37°C for 10 days after aging for 22 days at 30°C | Dual Agent Group-1 | 4.1 | 3.7 | 0 |
| | Dual Agent Group-2 | 4.1 | 3.7 | 0 |
| | Sole Agent Group | 4.4 | 2.9 | 0.1 |
| | Control Group | 4.9 | 1.6 | 0.6 |

It is seen from Table 1 that, as for aging at 30°C for 22 days, the test groups containing live bacteria-containing agents are excellent. Among the test groups containing live bacteria-containing agents, the dual agent groups -1 and -2 containing Streptococcus lactis C-6513 besides Lactobacillus casei C-1631 is better than the sole agent group containing only Lactobacillus casei C-1631. As for further aging at 37°C for ten days, the pH value and the amount of butyric acid are undesirably increased in the control group, whereas such phenomenon was scarcely noticed in the sole agent group or in the dual agent groups -1 and -2. Comparing the sole agent group and the dual agent groups -1 and -2 to each other, a small amount of butyric acid is seen to be produced in the sole agent group, whereas deterioration such as the formation of butyric acid is scarcely noticed in the dual agent groups -1 and -2, thus demonstrating that the high quality silage may be prepared during hotter seasons.

Panel tests by four panellers indicated that the sole agent and dual agent groups -1 and -2 were better than the control group under any aging conditions with respect to the flavor and tint. The dual agent groups -1 and -2 were superior to the sole agent group. In this manner, silage of superior quality could be prepared under both of the aging conditions.

Example 2

Reaped orchard grass were dried for half a day in the sun and shredded to give half dried grass. To 1.5kg of the produced half dried grass was added 0.75g of each of the live bacteria-containing agents obtained in Synthesis Examples 4 and 5 by way of preparing a dual agent group -1. Also, 1.5g of the live bacteria-containing agent prepared in the Synthesis Example 6 was added to the same amount of the half dried grass by way of preparing a dual agent group -2. These groups were packed under pressure in a polyethylene bag prepared from a thicker polyethylene sheet and aged at 25°C for 23 days to produce silage. After test samples were taken out, the silage was further aged at 37°C for 14 days. By way of preparing a sole agent group, 0.75g of the live bacteria-containing agent obtained in Synthesis Example 4 was added to the same amount of the half dried grass and aging was performed in the same way as in the dual agent groups. For comparison, the half dried grass not admixed with the live bacteria-containing agent was prepared as a control group.

The results are shown in Table 2.

The methods for preparation and analysis of the samples are the same as those shown in Example 1.

Table 2

| Aging Conditions | | pH |
|---|---|---|
| At 25°C for 23 days | Dual Agent Group-1 | 5.1 |
| | Dual Agent Group-2 | 5.0 |
| | Sole Agent Group | 5.2 |
| | Control Group | 5.4 |
| At 37°C for 14 days after aging for 23 days at 25°C | Dual Agent Group-1 | 4.9 |
| | Dual Agent Group-2 | 4.9 |
| | Sole Agent Group | 5.0 |
| | Control Group | 5.4 |

It is seen from the above Table 2 that, under both of the aging conditions, the test groups containing the live bacteria-containing agents are lower in pH values and excellent in flavor and tint, and that, among the test groups containing the live bacteria-containing agents, the dual agent groups containing the strain Streptococcus lactis C-6513 besides the strain Lactobacillus casei C-1631 is lower in pH and slightly better in flavor and tint than the sole agent group only containing the strain Lactobacillus casei C-1631.

Example 3

Reaped alfalfa was dried for half a day in the sun and shredded. By way of a sole live bacteria-containing agent group, 57.5g of the live bacteria-containing agent produced in the Synthesis Example 4 was added to 115kg of the half dried alfalfa and the resulting matrerial was packed under pressure into a 200 ℓ capacity drum can of the sealed type and aged for 28 days to produce silage. By way of preparing a dual agent group, the above sole agent group was admixed with 57.5g of the live bacteria-containing agent prepared in the Synthesis Example 5 and the resulting mixture was added to the half dried alfalfa in the similar manner as described above. The water contents in the packed alfalfa were 74.9 wt.% and the average temperature during the aging period was approximately 20°C. For comparison, the half dried alfalfa not admixed with the live bacteria-containing agent was also prepared as the control group.

The results are shown in Table 3.

The methods for preparation and analysis are the same as those of the Example 1. Evaluation of the produced grass silage was made on the basis of 40 points for panel tests, 60 points for pH and 100 points for organic acids, in accordance with the Frieg's evaluating system, with the 200 points as the full marks.

10

Table 3

| | Panel Test | | | | | pH | | Composition of Organic Acids | | | | | Total |
|---|---|---|---|---|---|---|---|---|---|---|---|---|---|
| | Flacor | Taste | Color | Feel | Points | pH | Points | Lactic Acid | Acetic Acid | Butyric Acid | Total Acid | Points | |
| Dual Agent Group | 7 | 8 | 7 | 6 | 28 | 4.48 | 24 | 5.091 | 0.854 | 0.079 | 6.024 | 100 | 152 |
| Sole Agent Group | 6 | 7 | 7 | 6 | 26 | 4.54 | 19 | 4.068 | 0.688 | 0.057 | 4.813 | 100 | 145 |
| Control Group | 5 | 7 | 7 | 6 | 25 | 4.58 | 15 | 4.627 | 0.834 | 0.065 | 5.526 | 99 | 139 |

It is seen from Table 3 that the silage of higher quality may be produced with the use of the live bacteria-containing agents and that, referring to the live bacteria-containing groups, the dual agent group containing the strain Streptococcus lactis C-6513 besides the strain Lactobacillus casei C-1631 is superior to the sole agent group containing only the strain Lactobacillus casei C-1631.

Example 4

For investigating the preservability of lactic acid bacteria in the live bacteria-containing agents obtained by the Synthesis Examples, test runs on survival rates of the lactic acid bacteria in the live bacteria-containing agents were conducted by allowing the agents to stand at 45°C for ten days. The results are shown in Table 4.

Table 4

| Synthesis Ex. No. | Number of Live Bacteria (Before test) | Number of Live Bacteria (After test) | Survival Rate (%) |
|---|---|---|---|
| 10-1 | $8 \times 10^8$/g | Lower than $1 \times 10^7$/g | Lower than 2 |
| 10-2 | $8 \times 10^8$/g | Lower than $1 \times 10^7$/g | Lower than 2 |
| 11 | $9 \times 10^8$/g | $6 \times 10^8$/g | 67 |
| 12 | $8 \times 10^8$/g | Lower than $1 \times 10^7$/g | Lower than 2 |
| 13 | $8 \times 10^8$/g | $4 \times 10^8$/g | 50 |
| 14 L.casei | $8 \times 10^8$/g | Lower than $1 \times 10^7$/g | Lower than 2 |
| S.lactis | $4 \times 10^8$/g | Lower than $1 \times 10^7$/g | Lower than 3 |
| 15 L.casei | $9 \times 10^8$/g | $5 \times 10^8$/g | 56 |
| S.lactis | $5 \times 10^8$/g | $3 \times 10^8$/g | 60 |

It is seen from the above results that the Synthesis Examples 11, 13 and 15 employing the corn grits pursuant to the present invention were improved significantly in survival rate as compared with the respective control synthesis examples employing the commercially available products.

Example 5

Using the live bacteria containing-agents produced in the Synthesis Example 10-2 and the Synthesis Example 11, silage of the scale of 10 tons of alfalfa was produced. The amounts of addition of the live bacteria-containing agents were set to 2 kg each. A perforated can was hung in a transport passage of the shredded grass and filled with the live bacteria-containing agents so as to be dripped continuously little by little onto the shredded grass. After fermentation for 1 month, samples were collected at three points, namely at upper, intermediate and lower portions of the silo and measured for pH values. The results are shown in Table 5.

Table 5

| Synthesis Ex. No. | Upper | pH Intermediate | Lower |
|---|---|---|---|
| No. Addition | 6.0 | 5.8 | 6.1 |
| 10-2 | 5.2 | 5.9 | 5.1 |
| 11 | 5.3 | 5.0 | 5.2 |

It is seen from the above results that, in the synthesis example 11 employing the corn grits of the present invention as the base, the pH value is lower than in the case of not adding the agent, for any of the three sampling points, thus demonstrating the favorable effect accrued from the addition of the live bacteria-containing agents. However, in the synthesis example 10-2, the effect of addition is non-uniformly changed from one sampling point in the silo to another. This may be explained in such a manner that the live bacteria-containing agent produced in the Synthesis Example 11 has improved fluidity and may be added to the grass substantially uniformly, whereas the live bacteria-containing agent produced in the synthesis example 10-2 lacks in fluidity and may not be added uniformly to the grass.

**Claims**

1. A lactic acid bacteria starter for preparation of silage comprising a strain Lactobacillus casei C-1631 (Deposit No. FERM BP-2260) having the following bacteriological properties as the effective bacteria:

(a) Bacteriological properties of Lactobacillus casei C-1631;

(1) gram stain: positive

(2) spore formation: -

(3) morphology: bacillus

(4) $CO_2$ production from glucose: -

(5) catalase production: -

(6) nitrate reducibility:-

(7) liquefaction of gelatine:-

(8) $H_2S$ production: -

(9) indole production: -

(10) aerobic growth: +

(11) growth at 15°C: +

(12) growth at 45°C: +

(13) optical activity of lactic acid: L (+)

(14) fermentation of carbohydrate

arabinose: -

xylose: -

rhamnose: -

glucose: +

mannose: +

fructose: +

galactose: +

sucrose: +

maltose: +

cellobiose: +

lactose: -

trehalose: +

melibiose: -

raffinose: -

starch: -

mannitol: +

sorbitol: +

esculin: +

salicin: +

2. The lactic acid bacteria starter for preparation of silage according to claim 1 wherein an initial pH of a medium cultivating said effective bacteria is 5 to 8.

3. The lactic acid bacteria starter for preparation of silage according to claim 1 wherein an incubation temperature of said effective bacteria is 20 to 42°C.

4. The lactic acid bacteria starter for preparation of silage according to claim 1 wherein an incubation time of said effective bacteria is 12 hours to three days.

5. A live bacteria-containing agent comprising the lactic acid bacteria starter for preparation of silage according to claim 1 and a base containing not less than 50 wt.% of corn grits having not more than 4 wt.% of water and a particle size of 10 to 80 meshes.

6. The live bacteria-containing agent according to claim 5 wherein said base contains less than 50 wt. % of an additive selected from the group consisting of calcium carbonate, zeolite, corn cob, dextrin and a mixture thereof.

7. A lactic acid bacteria starter for preparation of silage comprising a strain Lactobacillus casei C-1631 (Deposit No. FERM BP-2260) and a strain Streptococcus lactis C-6513 (FERM BP-2261) each having the following bacteriological properties as the effective bacteria:

(a) Bacteriological properties of Lactobacillus casei C-1631;

(1) gram stain: positive

(2) spore formation: -

(3) morphology: bacillus

(4) $CO_2$ production from glucose: -

(5) catalase production: -

(6) nitrate reducibility:-

(7) liquefaction of gelatine:-

(8) $H_2S$ production: -

(9) indole production: -

(10) aerobic growth: +

(11) growth at 15°C: +

(12) growth at 45°C: +

(13) optical activity of lactic acid: L (+)

(14) fermentation of carbohydrate

arabinose: -

xylose: -

rhamnose: -

glucose: +

mannose: +

fructose: +

galactose: +

sucrose: +

maltose: +

cellobiose: +

lactose: -

trehalose: +

melibiose: -

raffinose: -

starch: -

mannitol: +

sorbitol: +

esculin: +

salicin: +

(b) Bacteriological properties of Streptococcus lactis C-6513;

(1) gram stain: positive

(2) spore formation: -

(3) morphology: coccus

(4) $CO_2$ production from glucose: -

(5) catalase production: -

(6) growth on 0.1% methylene blue milk: +

(7) growth on 6.5% NaCl: -

(8) growth on 40% bile: +

(9) growth at pH of 9.6:-

(10) viability after processing at 60°C for 30 min: -

(11) hemolytic properties for horse's blood: -

(12) liquefaction of gelatine: -

(13) hydrolysis of starch: -

(14) decomposition of sodium hippurate: -

(15) hydrolysis of esculin: -

(16) $NH_3$ formation from arginine: +

(17) growth at 10°C: +

(18) growth at 45°C: -

(19) fermentation of carbohydrate;

arabinose: +

xylose: +

rhamnose: -

glucose: +

mannose: +

fructose: +

galactose: +

14

sucrose: +

maltose: +

cellobiose: +

lactose: +

trehalose: +

melibiose: -

raffinose: -

mannitol: +

sorbitol: -

salicin: +

8. The lactic acid bacteria starter for preparation of silage according to claim 7 wherein said strain Lactobacillus casei C-1631 (FERM BP-2260) and said strain Streptococcus lactis C-6513 (FERM BP-2261) as the effective bacteria are cultivated simultaneously.

9. The lactic acid bacteria starter for preparation of silage according to claim 7 wherein said strain Lactobacillus casei C-1631 (FERM BP-2260) and said strain Streptococcus lactis C-6513 (FERM BP-2261) as the effective bacteria are cultivated separately and subsequently mixed together.

10. The lactic acid bacteria starter for preparation of silage according to claim 7 wherein an initial pH of the medium cultivating said effective bacteria is 5 to 8.

11. The lactic acid bacteria starter for preparation of silage according to claim 7 wherein an incubation temperature of said effective bacteria is 20 to 42°C.

12. The lactic acid bacteria starter for preparation of silage according to claim 7 wherein an incubation time of said effective bacteria is 12 hours to three days.

13. A live bacteria-containing agent comprising the lactic acid bacteria starter for preparation of silage according to claim 7 and a base containing not less than 50 wt.% of corn grits having not more than 4 wt.% of water and a particle size of 10 to 80 meshes.

14. The live bacteria-containing agent according to claim 13 wherein said base contains less than 50 wt.% of an additive selected from the group consisting of calcium carbonate, zeolite, corn cob, dextrin and a mixture thereof.

**Patentansprüche**

1. Milchsäurebakterienstarter zur Herstellung von Silage, der einen Stamm Lactobacillus casei C-1631 (Hinterlegungsnummer FERM BP-2260) mit den nachfolgenden bakteriologischen Eigenschaften als wirksame Bakterien aufweist:

(a) bakteriologische Eigenschaften von Lactobacillus casei C-1631:

(1) Gram-Färbung: positiv

(2) Sporenbildung: -

(3) Morphologie: Bazillus

(4) $CO_2$-Erzeugung aus Glucose: -

(5) Katalaseerzeugung: -

(6) Nitratreduzierbarkeit: -

(7) Verflüssigung von Gelatine: -

(8) $H_2S$-Erzeugung: -

(9) Indolerzeugung: -

(10) aerobisches Wachstum: +

(11) Wachstum bei 15°C: +

(12) Wachstum bei 45°C: +

(13) optische Aktivität der Milchsäure: L (+)

(14) Fermentation von Carbohydrat

Arabinose: -

Xylose: -

15

Rhamnose: -

Glucose: +

Mannose: +

Fructose: +

Galactose: +

Sucrose: +

Maltose: +

Cellobiose: +

Lactose: -

Trehalose: +

Melibiose: -

Raffinose: -

Stärke: -

Mannitol: +

Sorbitol: +

Äskulin: +

Salicin: +

2. Milchsäurebakterienstarter zur Herstellung von Silage nach Anspruch 1, bei dem ein Ausgangs-pH-Wert eines Mediums, das die wirksamen Bakterien kultiviert, 5 bis 8 beträgt.

3. Milchsäurebakterienstarter zur Herstellung von Silage nach Anspruch 1, bei dem eine Inkubationstemperatur der wirksamen Bakterien 20 bis 42 °C beträgt.

4. Milchsäurebakterienstarter zur Herstellung von Silage nach Anspruch 1, bei dem eine Inkubationszeit der wirksamen Bakterien 12 h bis 3 Tage beträgt.

5. Lebende Bakterien enthaltendes Mittel, das den Milchsäurebakterienstarter zur Herstellung von Silage nach Anspruch 1 und eine Basis umfaßt, die nicht weniger als 50 Gewichtsprozent Getreideschrot mit nicht mehr als 4 Gewichtsprozent Wasser und einer Partikelgröße von 10 bis 80 mesh (2,00-0,18 mm) enthält.

6. Lebende Bakterien enthaltendes Mittel nach Anspruch 5, bei dem die Basis weniger als 50 Gewichtsprozent eines Additives enthält, das aus der Gruppe ausgewählt ist, die aus Kalziumcarbonat, Zeolith, Maiskolben, Dextrin und Gemischen davon besteht.

7. Milchsäurebakterienstarter zur Herstellung von Silage, der einen Stamm Lactobacillus casei C-1631 (Hinterlegungsnummer FERM BP-2260) und einen Stamm Streptococcus lactis C-6513 (FERM BP 2261), die jeweils die nachfolgenden bakteriologischen Eigenschaften besitzen, als wirksame Bakterien aufweist:

   (a) bakteriologische Eigenschaften von Lactobacillus Casei C-1631:

   (1) Gram-Färbung: positiv

   (2) Sporenbildung: -

   (3) Morphologie: Bazillus

   (4) $CO_2$-Erzeugung aus Glucose: -

   (5) Katalaseerzeugung: -

   (6) Nitratreduzierbarkeit: -

   (7) Verflüssigung von Gelatine: -

   (8) $H_2S$-Erzeugung: -

   (9) Indolerzeugung: -

   (10) aerobisches Wachstum: +

   (11) Wachstum bei 15 °C: +

   (12) Wachstum bei 45 °C: +

   (13) optische Aktivität der Milchsäure: L (+)

   (14) Fermentation von Carbohydrat

   Arabinose: -

   Xylose: -

   Rhamnose: -

Glucose: +

Mannose: +

Fructose: +

Galactose: +

Sucrose: +

Maltose: +

Cellobiose: +

Lactose: -

Trehalose: +

Melibiose: -

Raffinose: -

Stärke: -

Mannitol: +

Sorbitol: +

Äskulin: +

Salicin: +

(b) bakteriologische Eigenschaften von Streptococcus lactis C-6513:

(1) Gram-Färbung: positiv

(2) Sporenbildung: -

(3) Morphologie: Coccus

(4) $CO_2$-Erzeugung aus Glucose: -

(5) Katalaseerzeugung: -

(6) Wachstum auf 0,1% Methylenblaumilch: +

(7) Wachstum auf 6,5% NaCl:

(8) Wachstum auf 40% Galle: +

(9) Wachstum bei einem pH-Wert von 9,6: -

(10) Lebensfähigkeit nach Behandlung bei 60°C über 30 min: -

(11) hämolytische Eigenschaften für Pferdeblut: -

(12) Verflüssigung von Gelatine: -

(13) Hydrolyse von Stärke: -

(14) Zersetzung von Natriumhippurat: -

(15) Hydrolyse aus Äskulin: -

(16) $NH_3$-Erzeugung von Arginin: +

(17) Wachstum bei 10°C: +

(18) Wachstum bei 45°C: -

(19) Fermentation von Carbohydrat

Arabinose: +

Xylose: +

Rhamnose: -

Glucose: +

Mannose: +

Fructose: +

Galactose: +

Sucrose: +

Maltose: +

Cellobiose: +

Lactose: +

Trehalose: +

Melibiose: -

Raffinose: -

Mannitol: +

Sorbitol: -

Salicin: +

8. Milchsäurebakterienstarter zur Herstellung von Silage nach Anspruch 7, bei dem der Stamm Lactobacillus casei C-1631 (FERM BP-2260) und der Stamm Streptococcus lactis C-6513 (FERM BP-2261) als wirksame Bakterien gleichzeitig kultiviert werden.

9. Milchsäurebakterienstarter zur Herstellung von Silage nach Anspruch 7, bei dem der Stamm Lactobacillus casei C-1631 (FERM BP-2260) und der Stamm Streptococcus lactis C-6513 (FERM BP-2261) als wirksame Bakterien separat kultiviert und nacheinander gemischt werden.

10. Milchsäurebakterienstarter zur Herstellung von Silage nach Aspruch 7, bei dem ein Ausgangs-pH-Wert des die wirksamen Bakterien kultivierenden Mediums 5 bis 8 beträgt.

11. Milchsäurebakterienstarter zur Herstellung von Silage nach Anspruch 7, bei dem eine Inkubationstemperatur der wirksamen Bakterien 20 bis 42°C beträgt.

12. Milchsäurebakterienstarter zur Herstellung von Silage nach Anspruch 7, bei dem eine Inkubationszeit der wirksamen Bakterien 12 h bis 3 Tage beträgt.

13. Lebende Bakterien enthaltendes Mittel, das den Milchsäurebakterienstarter zur Herstellung von Silage nach Anspruch 7 und eine Basis aufweist, die nicht weniger als 50 Gewichtsprozent von Getreideschrot mit nicht mehr als 4 Gewichtsprozent Wasser und einer Partikelgröße von 10 bis 80 mesh (2,00-0,18 mm) enthält.

14. Lebende Bakterien enthaltendes Mittel nach Anspruch 13, bei dem die Basis weniger als 50 Gewichtsprozent eines Additives aufweist, das aus der Gruppe ausgewählt ist, die aus Kalziumcarbonat, Zeolith, Maiskolben, Dextrin und Gemischen davon besteht.

## Revendications

1. Culture bactérienne formant de l'acide lactique destinée à la préparation d'un ensilage comprenant, comme bactéries actives, une souche de Lactobacillus casei C-1631 (n° de dépôt FERM BP-2260) possédant les propriétés bactériologiques suivantes :
    (a) propriétés bactériologiques de Lactobacillus casei C-1631 :
        (1) souche à Gram positif
        (2) formation de spores : -
        (3) morphologie : bacille
        (4) production de $CO_2$ à partir de glucose : -
        (5) production de catalase : -
        (6) pouvoir de réduction des nitrates : -
        (7) liquéfaction de la gélatine : -
        (8) production de $H_2S$ : -
        (9) production d'indole : -
        (10) croissance aérobie : +
        (11) croissance à 15°C : +
        (12) croissance à 45°C : +
        (13) activité optique de l'acide lactique : L (+)
        (14) fermentation de glucide :
        arabinose : -
        xylose : -
        rhamnose : -
        glucose : +
        mannose : +
        fructose : +
        galactose : +
        saccharose : +
        maltose : +
        cellobiose : +
        lactose : -
        tréhalose : +
        mélibiose : -
        raffinose : -
        amidon : -
        mannitol : +

18

sorbitol : +

esculine : +

salicine: +.

2. Culture bactérienne formant de l'acide lactique destinée à la prépartion d'un ensilage selon la revendication 1, dans laquelle le pH initial d'un milieu de culture desdites bactéries actives est compris entre 5 et 8.

3. Culture bactérienne formant de l'acide lactique destinée à la préparation d'un ensilage selon la revendication 1, dans laquelle la température d'incubation desdites bactéries actives est comprise entre 20 et 42°C.

4. Culture bactérienne formant de l'acide lactique destinée à la préparation d'un ensilage selon la revendication 1, dans laquelle la durée d'incubation desdites bactéries actives est comprise entre 12 h et 3 jours.

5. Agent contenant des bactéries vivantes, comprenant la culture bactérienne formant de l'acide lactique destinée à la préparation d'un ensilage selon la revendication 1 et une base contenant au moins 50 % en masse de gruau de céréales ayant au plus 4 % en masse d'eau et une dimension de particule comprise entre 10 et 80 mesh.

6. Agent contenant des bactéries vivantes selon la revendication 5, dans lequel ladite base contient moins de 50 % en masse d'un additif choisi dans le groupe constitué par le carbonate de calcium, une zéolite, un épi de mais, la dextrine et un de leurs mélanges.

7. Culture bactérienne formant de l'acide lactique destinée à la préparation d'un ensilage comprenant, comme bactéries actives, une souche de Lactobacillus casei C-1631 (n° de dépôt FERM BP-2260) et une souche de Streptococcus lactis C-6513 (FERM BP-2261) possédant chacune les propriétés bactériologiques suivantes :

　　(a) propriétés bactériologiques de Lactobacillus casei C-1631 :

　　　　(1) souche à Gram positif

　　　　(2) formation de spores : -

　　　　(3) morphologie : bacille

　　　　(4) production de $CO_2$ à partir de glucose : -

　　　　(5) production de catalase : -

　　　　(6) pouvoir de réduction des nitrates : -

　　　　(7) liquéfaction de la gélatine : -

　　　　(8) production de $H_2S$ : -

　　　　(9) production d'indole : -

　　　　(10) croissance aérobie : +

　　　　(11) croissance à 15°C : +

　　　　(12) croissance à 45°C : +

　　　　(13) activité optique de l'acide lactique : L (+)

　　　　(14) fermentation de glucide :

　　　　arabinose : -

　　　　xylose : -

　　　　rhamnose : -

　　　　glucose : +

　　　　mannose : +

　　　　fructose : +

　　　　galactose : +

　　　　saccharose : +

　　　　maltose : +

　　　　cellobiose : +

　　　　lactose : -

　　　　tréhalose : +

　　　　mélibiose : -

　　　　raffinose : -

EP 0 329 164 B1

amidon : -
mannitol : +
sorbitol : +
esculine : +
salicine : +.

(b) propriétés bactériologiques de Streptococcus lactis C-6513 :
(1) souche à Gram positif
(2) formation de spores : -
(3) morphologie : coque
(4) production de $CO_2$ à partir de glucose : -
(5) production de catalase : -
(6) croissance dans du lait contenant 0,1 % de bleu de méthylène : +
(7) croissance dans du NaCl à 6,5 % : -
(8) croissance dans de la bile à 40% : +
(9) croissance à pH de 9,6 : -
(10) viabilité après traitement à 60 °C pendant 30 min : -
(11) propriétés hémolytiques sur le sang de cheval : -
(12) liquéfaction de la gélatine : -
(13) hydrolyse de l'amidon : -
(14) décomposition de l'hippurate de sodium : -
(15) hydrolyse de l'esculine : -
(16) formation de $NH_3$ à partir d'arginine : +
(17) croissance à 10 °C : +
(18) croissance à 45 °C : -
(19) fermentation de glucide :
arabinose : +
xylose : +
rhamnose : -
glucose : +
mannose : +
fructose : +
galactose : +
saccharose : +
maltose : +
cellobiose : +
lactose : +
tréhalose : +
mélibiose : -
raffinose : -
mannitol : +
sorbitol : -
salicine : +.

**8.** Culture bactérienne formant de l'acide lactique destinée à la préparation d'un ensilage selon la revendication 7, dans laquelle on cultive simultanément ladite souche de Lactobacillus casei C-1631 (FERM BP-2260) et ladite souche de Streptococcus lactis C-6513 (FERM BP-2261) comme bactéries actives.

**9.** Culture bactérienne formant de l'acide lactique destinée à la préparation d'un ensilage selon la revendication 7, dans laquelle on cultive séparément, puis on mélange ladite souche de Lactobacillus casei C-1631 (FERM BP-2260) et ladite souche de Streptococcus lactis C-6513 (FERM BP-2261) comme bactéries actives.

**10.** Culture bactérienne formant de l'acide lactique destinée à la préparation d'un ensilage selon la revendication 7, dans laquelle le pH initial du milieu de culture desdites bactéries actives est compris entre 5 et 8.

20

**11.** Culture bactérienne formant de l'acide lactique destinée à la préparation d'un ensilage selon la revendication 7, dans laquelle la température d'incubation desdites bactéries actives est comprise entre 20 et 42°C.

**12.** Culture bactérienne formant de l'acide lactique destinée à la préparation d'un ensilage selon la revendication 7, dans laquelle la durée d'incubation desdites bactéries actives est comprise entre 12 h et 3 jours.

**13.** Agent contenant des bactéries vivantes, comprenant la culture bactérienne formant de l'acide lactique destinée à la préparation d'un ensilage selon la revendication 7 et une base contenant au moins 50 % en masse de gruau de céréales ayant au plus 4 % en masse d'eau et une dimension de particule comprise entre 10 et 80 mesh.

**14.** Agent contenant des bactéries vivantes selon la revendication 13, dans lequel ladite base contient moins de 50 % en masse d'un additif choisi dans le groupe constitué par le carbonate de calcium, une zéolite, un épi de maïs, la dextrine et un de leurs mélanges.